# EUROPEAN PATENT APPLICATION

(11) **EP 3 915 416 A1**
(43) Date of publication of application: **01.12.2021**
(21) Application number: 21754881.7
(22) Date of filing: 15.03.2021
(51) Int. Cl.: A41D 31/04, A41B 17/00, A41B 9/06, A41D 13/00, A41D 27/08, A41D 31/00, A61F 5/01, C09J 183/04

(54) **ADHESIVE SILICONE PATTERN MATERIAL, AND WEAR COMPRISING SAME**

(30) Priority: 17.03.2020 KR 20200032586
(71) Applicant: Wavecompany Co., Ltd., Mapo-gu Seoul 03920 (KR)
(72) Inventor: LEE, Sang-Chul, Gangseo-gu, Seoul 07525 (KR); CHO, Na-Yun, Gangseo-gu, Seoul 07525 (KR)
(74) Representative: btb IP Bungartz Baltzer Partnerschaft mbB Patentanwälte
(86) International application number: PCT/KR2021/003163
(87) International publication number: WO 2021/187831

(57) **Abstract**

The present invention relates to an adhesive silicone patch to be applied to a wearable product. The adhesive silicone patch provides the function of assisting the musculoskeletal system and ligaments for each part of the human body, the function of preventing secondary damage, the function of supporting the injured part, and the function of promoting blood circulation. In addition, the adhesive silicone patch dramatically reduces the concentration of lactic acid generated during exercise. The present invention also relates to a wearable product provided with the adhesive silicone patch.

The adhesive silicone patch is applied to the inner surface of a wearable product such as clothing and includes a first functional layer.

The first functional layer includes a two-component liquid silicone rubber (LSR) and a silicone-based pressure-sensitive adhesive (PSA). The LSR is composed of a main component and a crosslinking agent. The PSA is included in an amount of 50 to 200 parts by weight with respect to 100 parts by weight of the LSR.

## Description

### Technical Field

The present invention relates to a functional patch and, more particularly, to an adhesive silicone patch that is formed on the inner surface of clothing to exert an excellent muscle taping effect, and a wearable product including the same.

### Background Art

Rehabilitative is a word derived from rehabilitation and is thus used to refer to rehabilitation. Based on this meaning, a rehabilitative tape refers to an adhesive tape that is attached to the body for the purpose of preventing, protecting, and treating injuries directly related to physical activities, and facilitating recovery of muscles and joints.

In general, as a professional treatment for orthopedic diseases such as sprains or damage to muscles or ligaments, a plaster cast or medial tape is used for fixation or reinforcement of the damaged muscle or ligament, or drug therapy or the like is used.

On the other hand, recently, as a treatment method for patients with muscle or ligament damage, the use of rehabilitative taping therapy to treat various diseases by fixing the muscles with a cloth tape with elasticity and adhesive strength to assist the contraction and relaxation of the muscles is increasing.

However, the treatment effect of the rehabilitative taping therapy, which is used for muscle pain, damage of ligaments such as sprains or strains, and muscle weakness, is obtained by accurate detection of the damaged area and precise taping based on the knowledge on the mechanical action of muscles and ligaments. Since rehabilitative taping therapy is a professional treatment method that needs to be performed in only hospitals or oriental clinics or by skilled specialists, it was difficult for the general public without specialized knowledge about muscles, ligaments, and joints to practice such taping therapy by themselves.

Therefore, even for minor muscle or ligament injuries, it is necessary for a patient to go to a hospital to receive treatment from a specialist. However, there are various problems such as the burden of medical expenses and commuting. Moreover, even though the taping treatment is performed by a specialist, there is a problem in that it is cumbersome and takes a lot of time because the tape must be torn into a pattern that varies depending on the shape and direction of the muscles and ligaments and be attached strip by strip.

As a solution to these problems, Korean Utility Model No. 0374353 discloses a rehabilitative tape set including at least two or more adhesive tapes having different lengths corresponding to respective parts of the body, in which each of the adhesive tapes is provided with or without one or more cutting lines depending on the part to which the adhesive tape is to be applied. However, the rehabilitative tape set has a problem in that a specific method for an accurate taping procedure according to the mechanical action of muscles and ligaments for each part of the human body is not provided. Therefore, the usability was not effective, and the treatment effect was questionable.

As an improvement to the above-stated technique, Korea Patent No. 0989829 discloses sports taping tights configured such that silicone urethane is locally superimposed on the inner surface of upper and lower portions of the tights so that the tension of the fabric is finely distributed and is largely increased without cutting the fabric. With such a configuration, the adhesion to the muscles of the body is maximized, and the force supporting the muscles and ligaments is enhanced when worn by a user. In addition, the tights have the advantage of protecting the human body by increasing exercise capability, protecting ligaments and muscles, and enhancing muscle strength.

However, the method disclosed in the patent document has a problem in that each part of the human body is related to the mechanical action of muscles and ligaments and is not related at all to the vascular circulation system. In addition, although the adhesion of silicone urethane to the muscle part of the human body is maximized by the tension of the fabric of the tights, there is a problem that the position of the silicone urethane patch is changed due to the movement of the human body or a gap occurs between the skin of the human body and the silicone urethane patch. Thus, the therapeutic effect is reduced.

On the other hand, during active physical activity such as exercise, glycogen, which makes energy in the body, is decomposed by the use of muscles, and as a result, lactic acid is produced, which makes people feel fatigued. Muscle taping has the effect of lowering the lactic acid generated during exercise. The reduction in the lactic acid in the body lowers the sensation of fatigue. For this reason, recently, there has been a demand for the development of a muscle taping technique that can further improve the lactic acid reduction performance.

### Disclosure

### Technical Problem

The present invention has been made to solve the above problems, and an objective of the present invention is to provide an adhesive silicone patch and a wearable product including the same, the adhesive silicone patch being capable of assisting the function of the musculoskeletal system and ligaments, preventing secondary damage to the musculoskeletal system and ligaments, supporting an injured site, enabling a smooth flow of blood circulation, and greatly improving the performance of reducing the concentration of lactic acid generated during exercise.

Another objective of the present invention is to provide an adhesive silicone patch and a wearable product including the same, the adhesive silicone patch being capable of significantly improving the function of the lower extremities, strongly compressing the lower extremities, and increasing proprioceptive sensation, compared to conventional patches or functional clothing such as knee protectors, tights, etc.

### Technical Solution

In order to accomplish the objectives of the present invention, there is provided an adhesive silicone patch to be provided on the inner surface of a piece of clothing, the member including a first functional layer and preferably further including a second functional layer.

The first functional layer includes: a two-component liquid silicone rubber (LSR) composed of a main component and a crosslinking agent; and a silicone-based pressure-sensitive adhesive (PSA) including polydimethylsiloxane (PDMS).

In addition, the silicone-based pressure-sensitive adhesive (PSA) is included in an amount of 50 to 200 parts by weight based on 100 parts by weight of the main component of the two-component liquid silicone rubber (LSR).

In addition, the main component of the two-component liquid silicone rubber (LSR) includes vinyl polydimethylsiloxane (vinyl PDMS), and a crosslinking agent of the two-component liquid silicone rubber (LSR) includes a silicon hydride (Si-H).

In addition, the second functional layer may be made of a material having elasticity, such as silicone, silicone rubber, rubber, or the like.

### Advantageous Effects

The adhesive silicone patch according to the present invention and the wearable product having the same have the advantages of effectively lifting and holding the skin according to the movement of the human body, thereby assisting the movement of the musculoskeletal system and ligaments for each part of the body, preventing secondary damage thereto, supporting injured parts, facilitating blood circulation, and reducing lactic acid generated during exercise.

The present invention provides the advantages that a taping position on a muscle does not change even with the movement of the human body and an excellent taping treatment effect can be obtained because there is no gap between the skin and the silicone patch when taped to the human body.

According to the present invention, when a clothing piece provided with the adhesive silicone patch is washed, the member is not easily peeled off or damaged. In addition, the surface of the adhesive silicone patch is dry cleaned or wiped with one of various commercially available cleaning agents such as wet tissues or alcohol or ethyl acetate, the adhesion of the member is easily recovered.

A knee protector to which the adhesive silicone patch of the present invention is attached has a lower extremity function improvement effect that is 8.5 times higher than that of commercially available existing knee protectors on the 5th day of wearing, and 10.34 times higher on the 5th day of wearing.

Tights to which the adhesive silicone patch of the present invention is attached exhibits 1.5 times better compression performance than existing tights to which a patch having no first functional layer is attached and 2 times better compression performance than commercially available common tights.

The adhesive silicone patch according to the present invention and the clothing having the same member have the effects of maximizing the improvement of the lower extremity function and the compression performance, compared to conventional patches or conventional functional clothing (for example, knee protector, tights, etc.), thereby improving proprioceptive sensation.

### Description of Drawings

- FIG. 1: is a schematic diagram illustrating a wearable product provided with an adhesive silicone patch according to one embodiment of the present invention;
- FIG. 2: is a cross-sectional view illustrating a wearable product according to one embodiment of the present invention, in which the adhesive silicone patch according to the present invention is formed on the inner surface of a wearable product;
- FIG. 3: is a cross-sectional view illustrating a wearable product according to a modification to one embodiment of the present invention, in which the adhesive silicone patch according to the present invention is formed on the inner surface of the wearable product;
- FIG. 4: is a diagram illustrating a pattern shape of an adhesive silicone patch according to a first embodiment of the present invention;
- FIG. 5: is a diagram illustrating a pattern shape of an adhesive silicone patch according to a second embodiment of the present invention;
- FIG. 6: is a diagram illustrating a pattern shape of an adhesive silicone patch according to a third embodiment of the present invention;
- FIG. 7: shows data of a test of measuring change in concentration of lactic acid when patches of Example 1 of the present invention and Comparative Examples 1 to 3 are used;
- FIG. 8: is an official test report related to the test results of FIG. 7, the report describing the change in the concentration of lactic acid; and
- FIG. 9: is a questionnaire for evaluation of lower extremity function.

### [Explanation of Reference Numerals in the Drawings]

- 1:: Body part
- 10:: Wearable product
- 20:: Adhesive silicone patch
- 30:: First functional layer of adhesive silicone patch
- 40:: Second functional layer of adhesive silicone patch

### Best Mode

The terminology used herein is for the purpose of describing particular embodiments only and is not intended to limit the scope of the present invention. As used herein, the singular forms "a", "an", and "the" are intended to include the plural forms as well unless the context clearly indicates otherwise. It will be further understood that the terms "comprises", "includes", or "has" when used in this specification specify the presence of stated features, regions, integers, steps, operations, elements and/or components, but do not preclude the presence or addition of one or more other features, regions, integers, steps, operations, elements, components, and/or combinations thereof.

In addition, the term "on" or "above" means on or above an object and does not necessarily mean an upper position based on the direction of gravity. That is, the term "on" or "above" referred to in the present specification includes not only a case that an element is located on or under another element but also a case that one element is located in front or rear of another element.

Also, when a portion of a region, plate, or the like is referred to as being "on another portion or on top of another portion", it may be directly on, be in contact with, spaced from the other portion, or another portion may be interposed between them.

It is also to be understood that when one element is referred to herein as being "connected to" or "coupled to" another element, it may be connected or coupled directly to the other element or connected or coupled to the other element via a mediating element interposed therebetween, unless specifically stated otherwise.

In addition, terms used in the specification, "first", "second", etc., may be used to describe various components, but the components are not to be construed as being limited to the terms. These terms are used only for the purpose of distinguishing a component from another component.

Herein below, preferred embodiments, advantages, and features of the present invention will be described in detail with reference to the accompanying drawings.

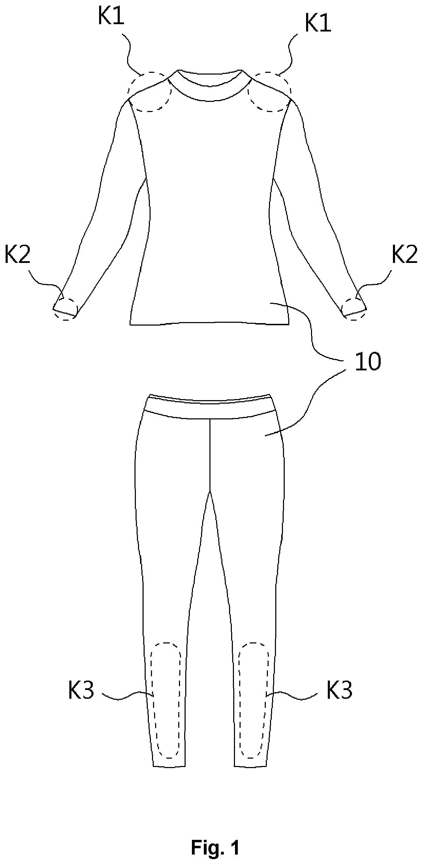

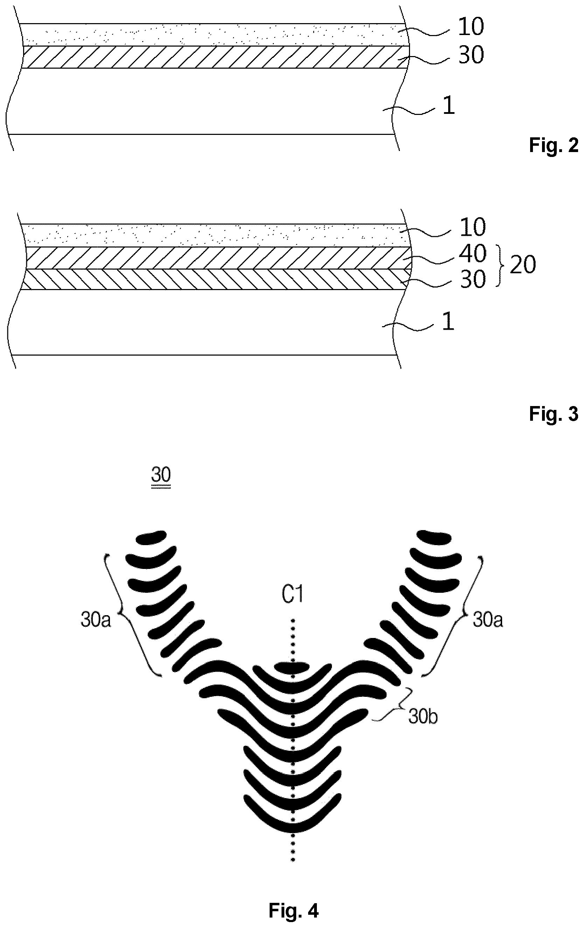

FIG. 1 is a schematic view of a wearable product provided with an adhesive silicone patch according to the present invention, and FIG. 2 is a cross-sectional view illustrating a state in which the adhesive silicone patch according to the present invention is formed on the inner surface of a wearable product.

Referring to FIGS. 1 and 2, an adhesive silicone patch 20 of the present invention has a predetermined shape and is formed on the inner side of a wearable product 10 such as clothing. The adhesive silicone patch 20 provides a muscle taping effect of holding and lifting the skin and functions to lower the concentration of lactic acid generated during exercise of the user of the wearing product 10.

The adhesive silicone patch 20 of the present invention may be applied to any position on the surface of the wearable product 10. For example, the adhesive silicone patch 20 may be attached to or integrally formed with at least one selected from among a shoulder region, a waist region, a wrist region, an elbow region, a calf region, and a knee region of the wearable product 10.

The inventor of the present invention found that, when an adhesive silicone patch having a predetermined shape and being made of silicone was formed on the inner surface of a wearable product, the adhesive silicone patch effectively lifted and held the skin even during the movement of the human body, thereby implementing the function of assisting musculoskeletal system and ligaments, preventing secondary damage, supporting the injured site, and promoting blood circulation. Thus, the inventor was granted a patent based on the finding under Korean Patent NO. 10-1268599.

Thereafter, the inventor of the present invention has found that when an adhesive silicone patch is prepared by mixing two-component liquid silicone lubber (LSR) and silicone-based pressure-sensitive adhesive (PSA) in a predetermined mixing ratio has the maximized effect of reducing the concentration of lactic acid generated during exercise as well as performs the functions described above.

The adhesive silicone patch 20 for a wearable product, according to the present invention, includes a first functional layer 30. Preferably, the adhesive silicone patch 20 additionally includes a second functional layer 40.

The first functional layer 30 of the adhesive silicone patch 20 is made of a mixture of a two-component liquid silicone rubber (LSR) and a silicone pressure-sensitive adhesive (PSA). Optionally, the mixture may further include a viscosity modifier.

The two-component liquid silicone rubber (LSR) of the present invention is a material that undergoes a crosslinking reaction at a high temperature of 100°C or higher and consists of two liquids that are respectively a main component and a crosslinking agent.

The main component and the crosslinking agent of the two-component liquid silicone rubber contain polydimethylsiloxane (PDMS) as a common component. The main component contains vinyl polydimethylsiloxane (vinyl PDMS), and the crosslinking agent has a structure having a silicon hydride (Si-H) group as a component corresponding to the vinyl PDMS.

The silicone-based pressure-sensitive adhesive (PSA) of the present invention is mixed with the two-component liquid silicone rubber to increase the adhesive force of the first functional layer 30 of the adhesive silicone patch 20 of the present invention. The silicone-based pressure-sensitive adhesive essentially includes polydimethylsiloxane (PDMS) and may optionally include a resin (for example, polytrimethylhydrosilyl silicate).

The silicone-based pressure-sensitive adhesive (PSA) is included in an amount of 50 to 200 parts by weight with respect to 100 parts by weight of the main component of the two-component liquid silicone rubber (LSR).

This is, when the mixing ratio of the silicone-based pressure-sensitive adhesive (PSA) is less than 50 parts by weight, the lactic acid reduction performance of the adhesive silicone patch 20 is as low as that of the conventional functional patch (refer to Comparative Example 1). On the other hand, when it exceeds 200 parts by weight, the wearer may suffer skin damage during intense exercise or undressing. For reference, the term "conventional functional patch" refers to a patch corresponding to Comparative Example 1 to be described later, and it corresponds to the patch disclosed in Korean Patent No. 10-1268599 invented by the same inventor of the present invention.

In the present invention, the viscosity modifier is added to the mixture consisting of the two-component liquid silicone rubber and the silicone-based pressure-sensitive adhesive to adjust the viscosity of the mixture. Preferably, silicone fluid may be used as the viscosity modifier.

In this case, the silicone fluid may be one or a mixture of at least two selected from among methylphenylsilicone fluid, alkylarylsilicone fluid, hydrogen silicon fluid, aminosilicone fluid, fluorosilicone fluid, and hydroxysilicone fluid.

An appropriate amount of the viscosity modifier may be optionally mixed as needed. When the viscosity modifier is added, preferably, the viscosity modifier may be added in an amount of 100 parts by weight or less with respect to 100 parts by weight of the main component of the two-component liquid silicone rubber (LSR). This is because when the mixing ratio of the viscosity modifier exceeds 100 parts by weight, the performance and durability of the first functional layer 30 are sharply decreased.

The first functional layer 30 of the adhesive silicone patch 20 of the present invention will be prepared in a manner disclosed below, by using a mixture of a main agent and a crosslinking agent of a two-component liquid silicone rubber, a silicone-based pressure-sensitive adhesive (PSA), and a viscosity modifier.

According to one preparation example, a mixture of a main agent of a two-component liquid silicone rubber, a crosslinking agent of the two-component liquid silicone rubber, a silicone-based pressure-sensitive adhesive (PSA), and a viscosity modifier is applied to the inner surface of a wearable product 10 such as clothing to form a predetermined pattern shape. Next, the applied mixture is heated to undergo a crosslinking reaction at a high temperature, so that the mixture is cured. In this case, the adhesive silicone patch 20 is integrated with the inner surface of the wearable product 10.

According to another preparation example, a mixture of a main agent of a two-component liquid silicone rubber, a crosslinking agent of the two-component liquid silicone rubber, a silicone-based pressure-sensitive adhesive (PSA), and a viscosity modifier may be printed on the inner surface of a wearable product 10 by a screen-printing method.

According to a further preparation example, a mixture of a main agent of a two-component liquid silicone rubber, a crosslinking agent of the two-component liquid silicone rubber, a silicone-based pressure-sensitive adhesive (PSA), and a viscosity modifier is poured into a mold having a predetermined pattern shape and is then pressurized at a high temperature. Thus, the adhesive silicone patch 20 having the desired pattern is produced.

According to a yet further preparation example, the adhesive silicone patch 20 may be configured in the form of a tape or sheet separate from the wearable product 10. In this case, for use of the adhesive silicone patch 20, the adhesive silicone patch 20 in the form of a tape or sheet may be attached to the inner surface of the wearable product 10.

When the adhesive silicone patch 20 is prepared in the form of a tape or sheet, the patch can be attached to the inner surface of the clothing 10 in various ways. For example, the first functional layer 30 of the adhesive silicone patch 20 or the second functional layer 40 to be described later may be attached to the inner surface of the clothing 10 by thermal fusion.

FIG. 3 is a cross-sectional view illustrating a wearable product according to a modification to one embodiment of the present invention, in which the adhesive silicone patch according to the present invention is formed on the inner surface of a wearable product.

Referring to FIG. 3, an adhesive silicone patch according to a modification to one embodiment of the present invention, additionally includes a second functional layer 40.

In the present invention, the second functional layer 40 functions to prevent muscle injury during exercise by increasing the tension of a specific region of the human body.

The second functional layer 40 is formed on a first surface of the first functional layer, the first surface being opposite to a surface to come into contact with the skin of the human body when the wearable product 10 is worn. That is, the second functional layer 40 is interposed between the wearable product 10 and the first functional layer 30.

The second functional layer 40 is made of an elastic material and, preferably, made of at least one material selected from silicone, silicone rubber, and rubber.

The adhesive silicone patch 20 prepared according to the above-described method may be formed in a suitable shape depending on a body part 1 to which the adhesive silicone patch 20 is to be applied. Preferably, the adhesive silicone patch 20 may be formed in a shape including a plurality of wave patterns.

In particular, the adhesive silicone patch 20 of the present invention is formed to have the composition and mixing ratio described above and is shaped to have wave patterns, thereby maximizing the effect of reducing the concentration of lactic acid.

In addition, when the adhesive silicone patch 20 is formed in a shape including wave patterns to be described later, advantages described below can be obtained. That is, when the patch 20 repeatedly comes into contact with a body part (for example, a joint such as an elbow or knee) where the skin is extremely stretched, it is possible to minimize the problem of skin troubles such as blisters caused by friction.

FIG. 4 is a diagram illustrating a pattern shape of an adhesive silicone patch according to a first embodiment of the present invention. According to the first embodiment, at least the first functional layer 30 of the adhesive silicone patch is disposed on the inner surface of a shoulder portion K1 of a top as shown in FIGS. 1 and 4, and the first functional layer 30 includes a plurality of wave patterns.

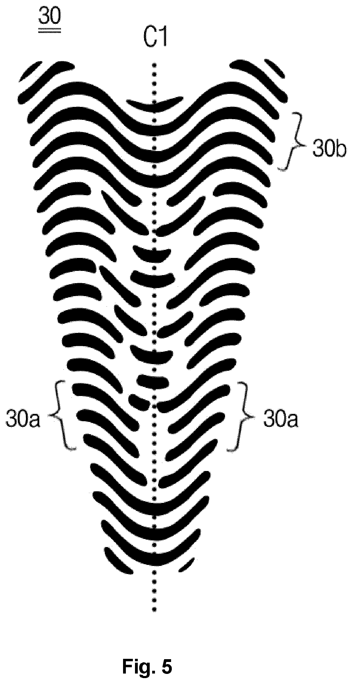

FIG. 5 is a diagram illustrating a pattern shape of an adhesive silicone patch according to a second embodiment of the present invention. According to the second embodiment, at least the first functional layer 30 of the adhesive silicone patch is disposed on the inner surface of a calf portion K3 of bottoms as shown in FIGS. 1 and 5, and the first functional layer 30 includes a plurality of wave patterns.

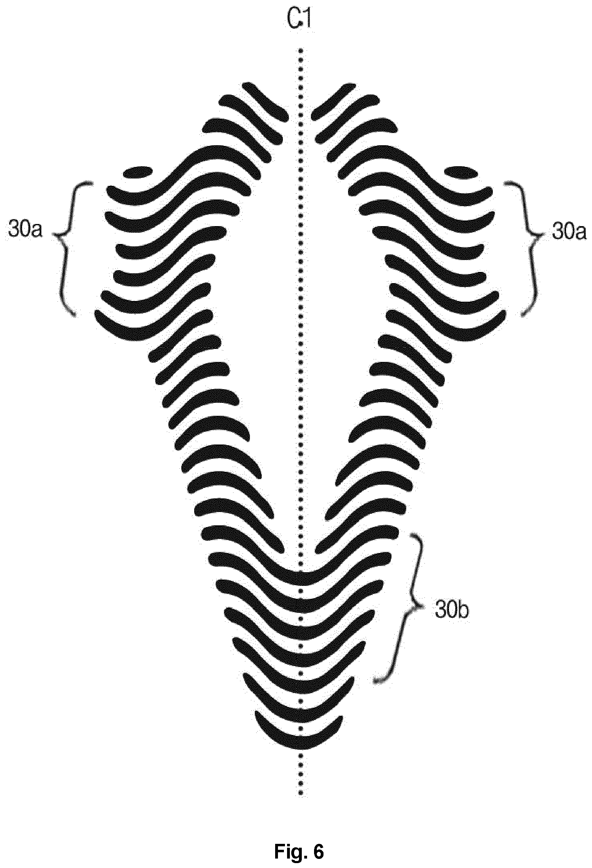

FIG. 6 is a diagram illustrating a pattern shape of an adhesive silicone patch according to a third embodiment of the present invention. According to the third embodiment, at least the first functional layer 30 of the adhesive silicone patch is disposed on the inner surface of a wrist portion K2 of a top as shown in FIGS. 1 and 6, and the first functional layer 30 includes a plurality of wave patterns.

According to a preferred embodiment, the first functional layer 30 of the adhesive silicone patch 20 has a shape in which multiple wave patterns are arranged at intervals in a longitudinal direction of the body part (for example, wrist, etc.) when the user wears the clothing (i.e., top or bottoms).

More preferably, the wave patterns of the first functional layer 30 of the adhesive silicone patch 20 are grouped into two or more pattern groups that are axial symmetric with respect to the central axis C1 extending in the longitudinal direction of the first functional layer 30. For example, reference numeral 30a in FIGS. 4, 5, and 6 denotes the symmetric pattern groups.

More preferably, the first functional layer 30 of the adhesive silicone patch 20 includes an expanding pattern group shape such that the width which is the size in a horizontal direction gradually increases from a lower end to an upper end thereof. For example, reference numeral 30b in FIGS. 4, 5, and 6 denotes the expanding pattern group. Here, the term "width" means a length of a linear line from the left end point to the right end point of a unit pattern in the pattern group. For example, reference numeral "W1" in FIG. 4 denotes the width (i.e., horizontal length) of the fourth unit pattern when counted from the bottom, among the unit patterns of the pattern group of FIG. 4.

The operational effects of the adhesive silicone patch 20 of the present invention described above will be described below.

When the wearable product (i.e., clothing) 10 on which the adhesive silicone patch 20 of the present invention is applied is worn, the first functional layer 30 of the adhesive silicone patch 20 elastically comes into contact with the skin 1 of the human body. Therefore, the skin 1 of the human body is subjected to a certain amount of pressure, and thus the first functional layer 30 of the adhesive silicone patch 20 attached to the inner surface of the wearable product (i.e., clothing) 10 becomes in tight contact with the targeted body part 1.

The adhesive silicone patch 20 attached to the skin of a body part 1 is wrinkled due to the movement of the body part 1. In this case, since the adhesive silicone patch 20 is in tight contact with or is adhesively attached to the skin, the skin is effectively lifted due to the wrinkles of the patch.

In this way, since the skin is lifted, a gap is created between the skin and the muscle, and the lymph fluid flows smoothly through this gap. In addition, the blood vessel expands and the blood flow in the blood vessel is facilitated.

Due to the above-described action of the adhesive silicone patch 20, that is, effectively holding and lifting of the skin during movement of the body part, the performance of reducing the lactic acid generated during the exercise of the wearer of the clothing 10 is improved.

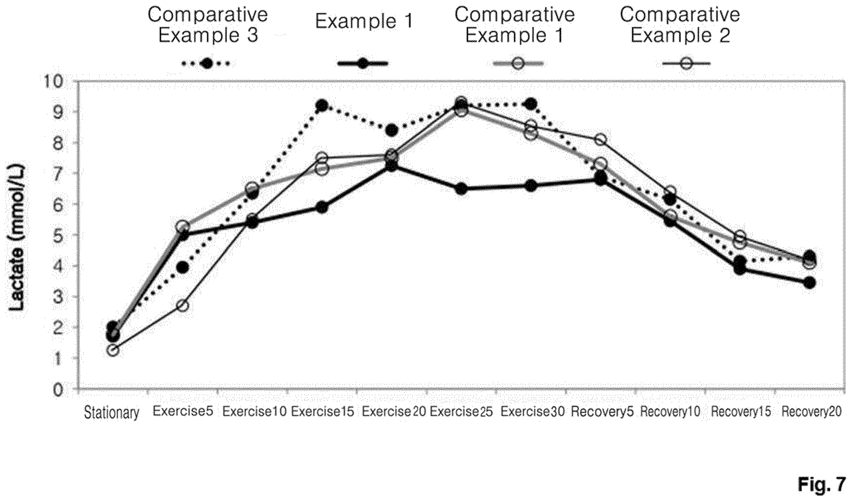

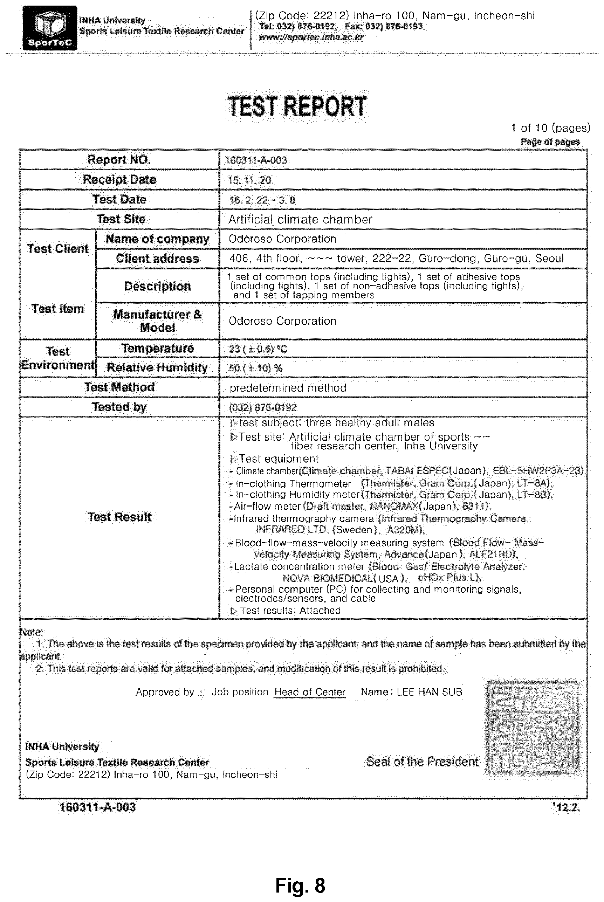

FIG. 7 is test result data showing change in concentration of lactic acid for patches of Example 1 and Comparative Examples 1 to 3, and FIG. 8 is an official test result certificate for the test results of FIG. 7.

Hereinafter, the lactic acid reduction performance of the adhesive silicone patches 20 of the present invention will be described in detail with reference to the test result data of the example and the comparative examples shown in FIG. 7. However, the examples are presented only for illustrative purposes and should not be interpreted to limit the scope of the present invention.

### [Example 1]

Adhesive silicone patches 20 are formed at various regions of upper and lower tights, the regions corresponding to the shoulders, waist, wrists, calves, and knees of the wearer, using a mixture of 100 parts by weight of a main component of a two-component liquid silicone rubber, 10 parts by weight of a crosslinking agent, 180 parts by weight of a silicone pressure sensitive adhesive (PSA), and 40 parts by weight of a silicone fluid.

### [Comparative Example 1]

Patches are formed at various regions of upper and lower tights, the regions corresponding to the shoulders, waist, wrists, calves, and knees of the wearer, using a mixture of 30 parts by weight of a viscosity modifier and 100 parts by weight of silicone.

### [Comparative Example 2]

Muscle sports tapes currently available on the market was purchased and taped to the shoulders, waist, wrists, calves, and knees of a user (i.e., test subject).

### [Comparative Example 3]

The same upper and lower tights as in Example 1 were used, but no patches were formed on or attached to the upper and lower tights. Taping therapy is not offered to the test subject.

### [Test Result 1]

Each of three healthy adult males exercised for 90 minutes, and the concentration of lactic acid was measured after the exercise. The 90-minute exercise was performed under each of four conditions by each male test subject. The conditions include wearing the tights of Example 1, wearing the tights of Comparative Example 1, wearing the taping of Comparative Example 2, and wearing the tights of Comparative Example 3.

The same participant took the 90-minute exercise in the same time zone on different days, while changing the tights or taping worn by the test subject during the exercise, among the tights and taping of Example 1 and Comparative Examples 1 to 3. The test results are sown in FIG. 7.

For reference, lactic acid is produced when glycogen, which makes energy in the body, is decomposed by the use of muscles, and is a substance that makes people feel fatigue. Therefore, when the concentration of lactic acid is reduced, the sensation of the fatigue is lowered.

Referring to FIG. 7, the lactic acid concentration of the test subject wearing the clothing of Example 1 on which the adhesive silicone patch 20 of the present invention is formed is always the lowest in a time zone from 10 minutes after the start of exercise to 20 minutes after recovery. The lactic acid concentration of the test subject wearing the clothing of Comparative Example 1 and the taping of Comparative Example 2 was intermediate, and the lactic acid concentration of the test subject wearing the clothing of Comparative Example 3 was the highest.

The test result data shows that the adhesive silicone patch 20 of the present invention exhibits a higher effect of reducing the concentration of lactic acid generated during exercise than the conventional patches or the conventional muscle taping.

That is, the adhesive silicone patch of the present invention maximizes the effect of reducing the concentration of lactic acid as shown by the test result 1 due to the organic combination of all of the factors including the composition, mixing ratio, and pattern shape.

The inventor of the present invention conducted lower extremity function evaluation and compression force evaluation described below to examine the lower extremity function improvement effect and the compression force of the adhesive silicone patch 20.

### (1) Lower Extremity (legs) Function Evaluation

Table 1 shows the results of evaluation of lower extremity.

**[Table 1]**

| Classification | Before wearing | Fifth day of wearing | Tenth day of wearing |
|---|---|---|---|
| Test group A | 64.0 | 67.4 | 72.3 |
| Comparative group A | 69.3 | 69.7 | 70.1 |

In Table 1, Experimental group A refers to a group in which test subjects wore a knee protector to which the adhesive silicone patch 20 of the present invention is attached, and Comparative group A refers to a group in which test subjects wore a general knee protector commercially available in the market. In Table 1, Experimental group A includes 10 people. The change in the lower extremity function was investigated in a manner that each test subject scores for each evaluation item on the day before wearing, the fifth day of wearing, and the tenth day of wearing.

In Table 1, Comparative group A includes 10 people. The change in the lower extremity function was investigated in a manner that each test subject scores for each evaluation item on the day before wearing, on the fifth day of wearing, and on the tenth day of wearing.

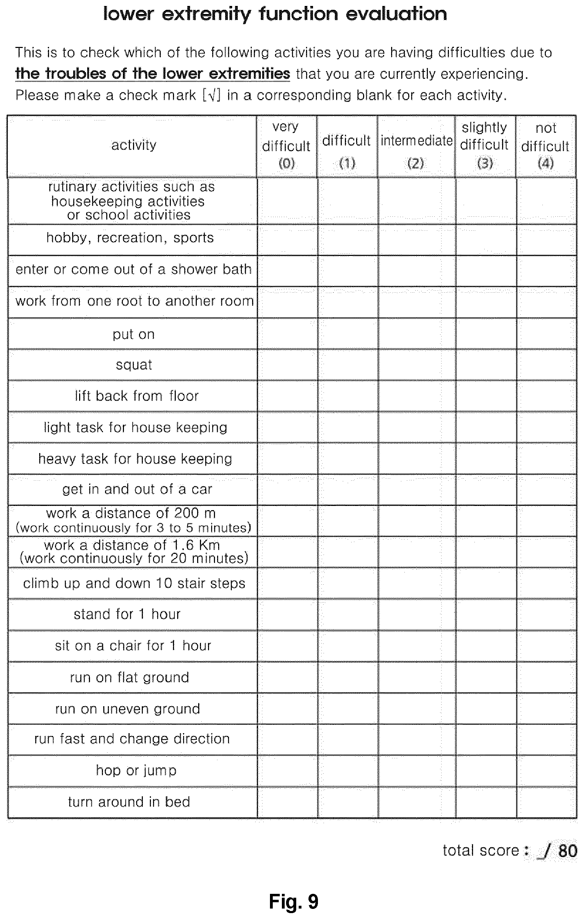

For reference, the survey shown in FIG. 9 was conducted in such a way that the subject made a check mark as the answer to each evaluation item (i.e., activity item) of the questionnaire, and a perfect score was set to 80 points.

In Table 1, the numerical values for "before wearing", "fifth day of wearing", and "tenth day of wearing" respectively, are the average values scored by the 10 people in Experimental group A and by the 10 people in Comparative group A in the questionnaire of FIG. 9.

Since the questionnaire in Table 1 is designed to record scores on a subjective basis for each person being investigated, the score for the item "before wearing" appears differently for each person being investigated. Accordingly, in Table 1, the score of the item "before wearing" in Experimental group A differs from the score of the item "before wearing" in Comparative group A.

Therefore, to find out the effect of improving the lower extremity function when wearing the knee protector, it is necessary to look at the amount of change (i.e., difference) in the score between the item "before wearing" and the item "after wearing" for each person investigated. The higher the change, the greater the improvement of the lower extremity function of the knee protector.

As can be seen from Table 1, in the case of Comparative Group A, the change in score between "before wearing" and "the fifth day of wearing" of the knee protector was 0.4, and the score change between "before wearing" and "the tenth day of wearing" was 0.8.

On the other hand, in the case of Test Group A, the change in score between "before wearing" and "the fifth day of wearing" of the knee protector was 3.4, and the score change between "before wearing" and "the tenth day of wearing" was 8.3.

That is, the knee protector to which the adhesive silicone patch of the present invention is attached has a lower extremity function improvement effect that is 8.5 times higher than that of the commercially available existing knee protector on the 5th day of wearing, and 10.34 times higher on the 5th day of wearing.

### (2) Evaluation of Compression Force

Table 2 shows the results of evaluation of compression force.

**[Table 2]**

| Classification | Standing posture (kPa) | Sitting posture (kPa) | Difference (kPa) |
|---|---|---|---|
| #1 | 1.21(±0.03) | 3.87(±0.01) | 2.66 |
| #2 | 0.89(±0.01) | 2.63(±0.00) | 1.74 |
| #3 | 2.24(±0.00) | 3.35(±0.00) | 1.11 |
| #4 | 4.15(±0.00) | 4.05(±0.00) | 0.10 |

In Table 2, "#1" denotes tights (wearable product) to which the adhesive silicone patch 20 of the present invention is attached. "#2"' denotes tights (wearable product) to which the patch that is not provided with the first functional layer 30 is attached. Specifically, the tights denoted by "#1" and "#2" are clothing with the model name "MEN TIGHTS L20" (model name) under the brand name "WaveWear". The tights denoted by "#3" are no-patched clothing with the model name "L-103-No.21" under the brand name "ArmorSkin". The tights denoted by "#4" are no-patched clothing with the model name "3ZK9L2WX00415" under the brand name "UnderArmour".

Table 2 shows the average value (± standard deviation) of 60 measurements of the compression force in units of kPa for each of "#1", "#2", "#3", and "#4". The compression force was measured at intervals of one second for 1 minute (i.e., 60 times of measurement for 1 minute) for each of a standing posture and a sitting posture. The item "difference (kPa)" among the items in Table 2 means the difference (kPa) in the compression force standing posture and the sitting posture.

To evaluate the compression performance in Table 2, it is necessary to look at the difference in the compression force (kPa) between the sitting posture and the standing posture. In other words, the higher the increase in the compression force when changing from the standing posture to the sitting posture, the better the compression performance is.

As can be seen from Table 2, the compression force difference of "#1" is 2.66, the compression force difference of "#2" is 1.74, the compression force difference "#3" is 1.11, and the compression force difference of "4" is 0.10. That is, the product corresponding to "#1'" among the products evaluated for compression force in Table 2 showed the best compression performance.

The tights to which the adhesive silicone patch of the present invention was attached exhibited 1.5 times better compression performance than existing tights to which a patch having no first functional layer 30 is attached and 2 times better compression performance than commercially available common tights.

### (3) Proprioceptive Sensation Improvement Effect

The effect of improving lower extremity function described above is related to the improvement of proprioceptive sensation. When the compression force and the kinetic taping effect are simultaneously expressed, good proprioceptive sensation is obtained. In this case, the higher the compression force, the more the proprioceptive sensation can be improved.

As can be seen from Tables 1 and 2, the adhesive silicone patch according to the present invention and the clothing having the same member have the effects of maximizing the improvement of the lower extremity function and the compression performance, compared to conventional patches or conventional functional clothing (for example, knee protector, tights, etc.), thereby improving proprioceptive sensation.

Although preferred embodiments of the present invention have been described and illustrated using specific terms, it is apparent that those terms are used only for clarification of the present invention but not for limiting the scope of the present invention. Accordingly, it is apparent that those embodiments and terms can be modified, changed, altered, and substitutes without departing from the technical spirit and scope of the present invention as defined in the appended claims. It should be noted that modifications and equivalents to the embodiments fall within the scope of the present invention.

## Claims

1. An adhesive silicone patch to be applied to an inner surface of a wearable product, the adhesive silicone patch comprising a first functional layer, wherein the first functional layer comprises: a two-component liquid silicone rubber (LSR) composed of a main component and a crosslinking agent; and a silicone-based pressure-sensitive adhesive (PSA) comprising polydimethylsiloxane (PDMS).

2. The adhesive silicone patch of claim 1, wherein the silicone-based pressure-sensitive adhesive (PSA) is included in an amount of 50 to 200 parts by weight based on 100 parts by weight of the main component of the two-component liquid silicone rubber (LSR).

3. The adhesive silicone patch of claim 1, wherein the main component of the two-component liquid silicone rubber (LSR) comprises vinyl polydimethylsiloxane (Vinyl PDMS), and the crosslinking agent of two-component liquid silicone rubber (LSR) comprises silicon hydride (Si-H).

4. The adhesive silicone patch of claim 1, wherein the first functional layer further comprises a viscosity modifier comprising a silicone fluid.

5. The adhesive silicone patch of claim 1, wherein the first functional layer includes a plurality of wave patterns arranged at intervals from each other, the plurality of wave patterns comprises symmetric pattern groups that are axially symmetric with respect to a central axis (C1) extending in a longitudinal direction, and each of the pattern groups is arranged such that a width that is a size in a horizontal direction gradually increases from a lower end portion to an upper end portion of each pattern group.

6. The adhesive silicone patch of claim 1, further comprising a second functional layer made of an elastic material and disposed on a first surface of the first functional layer, the first surface being opposite to a surface to come into contact with the skin.

7. A functional wearable product comprising:
a wearable product; and the adhesive silicone patch of any one of claims 1 to 6, the adhesive silicon patch being formed on the inner surface of the wearable product.
